# FASCICULE DE BREVET EUROPEEN

(11) **EP 3 169 654 B1**
(45) Date de publication et mention de la délivrance du brevet: **29.08.2018**
(21) Numéro de dépôt: 15727016.6
(22) Date de dépôt: 10.06.2015
(51) Int. Cl.: C07C 5/27, C07C 7/13, C07C 7/00, C07C 7/04, C07C 15/08, B01D 15/18

(54) **PROCÉDÉ DE PRODUCTION DE PARAXYLÈNE COMPRENANT DEUX UNITÉS DE SÉPARATION EN LIT MOBILE SIMULÉ ET DEUX UNITÉS D'ISOMÉRISATION DONT L'UNE EN PHASE GAZ**
VERFAHREN ZUR HERSTELLUNG VON PARAXYLEN, MIT ZWEI SIMULIERTEN WANDERBETTTRENNUNGSEINHEITEN UND ZWEI ISOMERISIERUNGSEINHEITEN, MIT EINER DAVON IN DER GASPHASE
METHOD FOR THE PRODUCTION OF PARAXYLENE, COMPRISING TWO SIMULATED MOVING BED SEPARATION UNITS AND TWO ISOMERISATION UNITS, ONE BEING IN THE GAS PHASE

(30) Priorité: 18.07.2014 FR 1456941
(43) Date de publication de la demande: 24.05.2017
(73) Titulaire: IFP Energies nouvelles, 92500 Rueil-Malmaison (FR)
(72) Inventeur: DREUX, Heloise, F-69007 Lyon (FR); LEFLAIVE, Philibert, F-69780 Mions (FR); LEINEKUGEL LE COCQ, Damien, F-69600 Oullins (FR)
(86) Numéro de dépôt international: PCT/EP2015/062984
(87) Numéro de publication internationale: WO 2016/008653

(56) Documents cités:
- FR-A1- 2 862 638
- US-A1- 2014 155 667

## Description

### DOMAINE DE L'INVENTION

La production de paraxylène est en constante augmentation depuis trente ans. Les utilisations principales du paraxylène sont les productions d'acide téréphtalique et de résines polyéthylène téréphtalate, pour fournir des textiles synthétiques, des bouteilles, et plus généralement des matières plastiques.

Pour satisfaire la demande toujours croissante en paraxylène, les pétrochimistes ont le choix entre pratiquer des augmentations de capacité sur des unités existantes ou construire des unités neuves.

La présente invention permet de répondre à ces deux cas de figure. Notamment elle permet de faire face à des augmentations de capacité d'unités existantes (appelées dégoulottage) car les modifications impliquées sont relativement modestes.

Dans la suite du texte on parle d'unité de séparation en lit mobile simulé (en abrégé SMB), ou d'unité de séparation (SMB). Une unité de séparation (SMB) peut contenir un ou plusieurs adsorbeurs.

### EXAMEN DE L'ART ANTERIEUR

La production de paraxylène haute pureté par séparation par adsorption est bien connue de l'art antérieur. De manière industrielle, cette opération est réalisée par un enchainement de procédés dit « boucle C8-aromatique ». Cette « boucle C8-aromatique » inclut une étape d'élimination des composés lourds (i.e. C9+) dans une colonne de distillation appelée « colonne des xylènes ». Le flux de tête de cette colonne, qui contient les isomères en C8-aromatiques, est ensuite envoyé dans le procédé de séparation du paraxylène qui est très généralement un procédé de séparation par adsorption en lit mobile simulé.

L'extrait, qui contient le paraxylène est ensuite distillé dans une colonne d'extrait puis une colonne dite « toluène » pour obtenir du paraxylène de haute pureté.

Le raffinat, riche en métaxylène, orthoxylène et éthylbenzène, après une étape d'élimination du solvant par distillation est traité dans une unité catalytique d'isomérisation qui redonne un mélange d'aromatiques en C8, dans lequel la proportion des xylènes (ortho-, méta-, para-xylènes) est pratiquement à l'équilibre thermodynamique, et la quantité d'éthylbenzène amoindrie. Ce mélange est à nouveau envoyé dans la « colonne des xylènes » avec la charge fraiche.

Tous les procédés industriels d'isomérisation des C8-aromatiques permettent d'isomériser les xylènes. La transformation de l'éthylbenzène dépend, en revanche, du type de procédé et de catalyseur choisis. En effet, les complexes pétrochimiques utiliseront une unité d'isomérisation dite « isomérisante » (i.e. isomérisant l'éthylbenzène en un mélange de C8 aromatiques) ou « désalkylante » (désalkylation de l'éthylbenzène en benzène), afin de privilégier la production respectivement soit de para-xylène seul, soit de benzène et para-xylène.

Le choix du catalyseur utilisé dépend de la transformation de l'éthylbenzène souhaitée. Lorsque la réaction cible est l'isomérisation de l'éthylbenzène, elle nécessite un catalyseur bifonctionnel présentant à la fois une fonction acide et une fonction hydrogénante. Il a en effet été démontré que l'éthylbenzène est tout d'abord hydrogéné en éthylcyclohexène sur les sites métalliques, puis transformé en dimethylcyclohexène sur sites acides par contraction puis expansion de cycle, et enfin deshydrogéné en xylènes.

Lorsque la réaction cible est la désalkylation de l'éthylbenzène, elle se produit uniquement sur des sites acides. La présence d'une phase hydrogénante sur le catalyseur permet cependant d'hydrogéner immédiatement l'éthylène formé et d'obtenir une désalkylation totale, évitant ainsi toute réalkylation ultérieure. Dans les deux cas, l'incorporation d'une phase métallique dans le catalyseur permet également d'assurer sa stabilité.

Les procédés industriels d'isomérisation utilisent donc des catalyseurs hétérogènes bifonctionnels (acides et métalliques) mis en oeuvre en lit fixe et opérant en phase vapeur sous pression d'hydrogène, dans des gammes de température comprises généralement entre 380-440°C et des pressions de 10 à 20 bar.

Le choix d'une isomérisation «isomérisante» permet, comme indiqué ci-dessus, de maximiser la production de paraxylène, qui est le composé ayant la plus forte valeur ajoutée en sortie du complexe aromatique. Cette solution présente cependant l'inconvénient de générer lors de l'étape d'isomérisation des pertes en cycles aromatiques par craquage plus importante qu'avec une isomérisation désalkylante, le cycle étant transitoirement au moins partiellement hydrogéné.
Le choix du type d'isomérisation est donc un compromis entre la minimisation de la perte de cycles aromatiques associée à une coproduction de benzène, produit à plus faible valeur ajoutée que le paraxylène (isomérisation désalkylante), et une maximisation de la production de paraxylène qui présente elle l'inconvénient de générer des pertes en cycles aromatiques plus importante (isomérisation « isomérisante »).
Il existe donc un besoin pour un procédé permettant à la fois une maximisation de la quantité de paraxylène produite avec une perte en cycles aromatiques réduite.
Plusieurs solutions sont proposées dans l'art antérieur afin d'atteindre cette objectif, celles-ci mettant généralement en oeuvre une isomérisation (généralement de préférence désalkylante), associée à des étapes de conversion du benzène par transalkylation et/ou méthylation du toluène ou du benzène, telles que décrites par exemple dans le document US2013/0267746.
Il a été découvert de manière surprenante que l'association au sein d'un complexe aromatique d'une isomérisation « isomérisante » et d'une isomérisation en phase liquide telle que décrite par exemple dans les brevets US2011/263918, US7,371,913, US4,962,258 et US6180550 permettait de maximiser la quantité de paraxylène produite tout en ayant une perte en cycles aromatiques réduite par rapport à un complexe aromatique selon l'art antérieur.
Dans la suite du texte on parle d'unité de séparation pour désigner les unités de séparation en lit mobile simulé, d'adsorbeurs pour désigner des ensembles de lits d'adsorbant, une unité pouvant contenir un ou plusieurs adsorbeurs.
On parle d'unité d'isomérisation et de colonnes à distiller pour désigner les autres équipements du procédé.

FR 2 862 638 A1 divulgue un procédé de production de paraxylène à partir d'une charge contentant des xylènes, de l'éthylbenzène et des hydrocarbures en C9+ comprenant une étape d'adsorption dans une unité opérant en lit mobile simulé et deux étapes d'isomérisation des aromatiques en C8.

US 2014/0155667 A1 divulgue un procédé de production de paraxylène à partir d'une charge contenant des aromatiques en C8+ qui utilise une unité de séparation (par exemple une unité Parex™) et deux unités d'isomérisation.

### DESCRIPTION SOMMAIRE DES FIGURES

La figure 1 est un schéma du procédé selon l'invention dans lequel on a fait apparaitre les deux unités de séparation en lit mobile simulé, notées (SMB-1) et (SMB-2), et les deux unités d'isomérisation notées (ISOM-1) et (ISOM-2).
La figure 2 représente une variante du procédé selon l'invention dans laquelle l'unité de séparation (SMB-1) utilise le PDEB comme désorbant et l'unité de séparation (SMB-2) utilise le toluène comme adsorbant. De ce fait, le toluène est récupéré en tête des colonnes à distiller (EXT-2) et (RAF-2), car le toluène est plus léger que les C8 aromatiques.
La figure 3 représente un schéma selon l'art antérieur et sert à illustrer l'exemple 1 qui est selon l'art antérieur.

### DESCRIPTION SOMMAIRE DE L'INVENTION

La présente invention peut se définir comme un procédé de production de paraxylène à haute pureté, à partir d'une coupe xylènes contenant de l'éthylbenzène et des composés en C9+.

Le procédé selon la présente invention utilise deux unités de séparation en lit mobile simulé (SMB-1 et SMB-2), et deux unités d'isomérisation (ISOM-1 et ISOM-2). Le dit procédé consiste en la suite d'étapes suivantes :
- on envoie la charge (2) dans une colonne de distillation (S-1), de laquelle on soutire en tête un mélange (3) comprenant la majeure partie du métaxylène, du paraxylène, de l'éthylbenzène, et au moins une partie de l'orthoxylène, et en fond un flux (4) d'hydrocarbures en C9-C10, et la partie restante de l'orthoxylène,
- on effectue une première séparation du mélange de tête (3) dans l'unité de séparation (SMB-1) comportant au moins un adsorbeur contenant une pluralité de lits interconnectés et travaillant en boucle fermée, ladite unité comprenant au moins quatre zones délimitées par les injections du flux (3) et du désorbant (10) résultant du mélange des flux (9) et (7), et les soutirages d'un premier extrait (5) enrichi en paraxylène, et d'un premier raffinat (8) appauvri en paraxylène,
- on effectue une deuxième séparation dans l'unité de séparation (SMB-2) de l'isomérat (12) issu de l'unité d'isomérisation (ISOM-1), ladite unité de séparation (SMB-2) étant constituée d'au moins un adsorbeur contenant une pluralité de lits interconnectés et travaillant préférentiellement en boucle fermée, et ladite unité comprenant au moins quatre zones délimitées par les injections de la charge (12) et du désorbant (18) résultant des flux (17) et (15), et les soutirages d'un deuxième extrait (13) enrichi en paraxylène, et d'un second raffinat (16) appauvri en paraxylène,
- on distille le premier extrait (5) issu de l'unité de séparation (SMB-1) dans une colonne à distiller (EXT-1), pour récupérer un flux (6) enrichi en paraxylène, et un flux (7) qui est utilisé comme désorbant de l'unité de séparation (SMB-1),
- on distille le second extrait (13) issu de l'unité de séparation (SMB-2) dans une colonne à distiller (EXT-2), pour récupérer un flux (14) enrichi en paraxylène, et un flux (15) qui est utilisé comme désorbant de l'unité de séparation (SMB-2),
- on distille le raffinat (16) issu de l'unité de séparation (SMB-2) dans une colonne de distillation (RAF-2), de manière à produire un flux (19) qui alimente l'unité d'isomérisation (ISOM-2), et un flux (17) qui est utilisé comme désorbant de l'unité de séparation (SMB-2),
- on distille le raffinat (8) issu de l'unité de séparation (SMB-1) dans une colonne à distiller (RAF-1) qui produit le flux (11) qui alimente l'unité d'isomérisation (ISOM-1), et un flux (9) qui est utilisé comme désorbant de l'unité de séparation (SMB-1),
- on alimente par le flux (11) l'unité d'isomérisation (ISOM-1) pour obtenir le premier isomérat (12),
- on alimente par le flux (19) la deuxième unité d'isomérisation (ISOM-2) pour obtenir un deuxième isomérat (20) qui est recyclé en entrée de la colonne à distiller (S-1), ladite unité d'isomérisation (ISOM-2) fonctionnant en phase gaz et aux conditions suivantes :
   - température supérieure à 300°C, de préférence de 350°C à 480°C,
   - pression inférieure à 4,0 MPa, et de préférence de 0,5 à 2,0 MPa,
   - vitesse spatiale inférieure à 10 h⁻¹, de préférence comprise entre 0,5 h⁻¹ et 6 h⁻¹,
   - rapport molaire hydrogène sur hydrocarbure inférieur à 10, et de préférence compris entre 3 et 6,
   et le catalyseur utilisé dans ladite unité d'isomérisation (ISOM-2) comportant au moins une zéolithe présentant des canaux dont l'ouverture est définie par un anneau à 10 ou 12 atomes d'oxygène (noté 10 MR ou 12 MR), et au moins un métal du groupe VIII de teneur comprise entre 0,1 et 0,3% poids, bornes incluses.

De façon préférée, dans le procédé de production de paraxylène à haute pureté selon l'invention, l'unité d'isomérisation (ISOM1) fonctionne en phase liquide aux conditions suivantes :
- Température inférieure à 300°C, de préférence comprise entre 200 et 260°C,
- Pression inférieure à 4 MPa, de préférence comprise entre 2 et 3 MPa,
- Vitesse spatiale inférieure à 10 h⁻¹, de préférence comprise entre 2 h⁻¹ et 4 h⁻¹,
- Catalyseur comportant au moins une zéolithe présentant des canaux dont l'ouverture est définie par un anneau à 10 ou 12 atomes d'oxygène (noté 10 MR ou 12 MR), préférentiellement une zéolithe présentant des canaux dont l'ouverture est définie par un anneau à 10 atomes d'oxygène (noté 10 MR), et de manière encore plus préférée une zéolithe de type ZSM-5.

De façon préférée, dans le procédé de production de paraxylène à haute pureté selon l'invention, le catalyseur utilisé pour l'unité d'isomérisation en phase gaz (ISOM-2) contient une zéolithe de type structural EUO ou MOR, et de manière préférée, une zéolithe EU-1 et du platine.

Selon une variante du procédé de production de paraxylène à haute pureté selon l'invention, l'unité de séparation (SMB-1) utilise comme désorbant le PDEB.

Selon une autre variante du procédé de production de paraxylène à haute pureté selon l'invention, l'unité de séparation (SMB-2) utilise comme désorbant le toluène.

Selon une variante du procédé de production de paraxylène à haute pureté selon l'invention, les unités de séparation (SMB-1) et (SMB-2) contiennent chacune de 6 à 24 lits, et de manière préférée de 8 à 15 lits répartis sur un ou plusieurs adsorbeurs, le nombre de lits étant ajusté de manière à ce que chaque lit ait, de préférence, une hauteur comprise entre 0,70 m et 1,40 m.

De façon préférée, dans le procédé de production de paraxylène à haute pureté selon l'invention, la répartition de la quantité de solide adsorbant dans les unités de séparation (SMB-1) et (SMB-2) est la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%,
les zones étant définies de la façon suivante :
- la zone 1 étant comprise entre l'injection du désorbant et le soutirage de l'extrait,
- la zone 2 étant comprise entre le soutirage de l'extrait et l'injection de la charge,
- la zone 3 étant comprise entre l'injection de la charge et le soutirage du raffinat,
- la zone 4 étant comprise entre le soutirage du raffinat et l'injection du désorbant.

Selon une autre variante préférée du procédé de production de paraxylène à haute pureté selon l'invention, en ce qui concerne l'unité de séparation (SMB-1), le rapport volumétrique désorbant sur charge est d'au plus 1,7/1, et de manière préférée compris entre 1,5/1 et 0,4/1, bornes comprises.

Selon une autre variante du procédé de production de paraxylène à haute pureté selon l'invention, en ce qui concerne l'unité de séparation (SMB-2), le rapport volumétrique désorbant sur charge est d'au plus 1,7/1, et de manière préférée compris entre 1,5/1 et 0,4/1 bornes comprises.

Un des avantages du procédé de production de paraxylène à haute pureté selon l'invention est qu'il permet de dégoullotter unité existante, constituée de deux adsorbeurs utilisés en série, de la manière suivante :
- on connecte le dernier lit du premier adsorbeur au premier lit du premier adsorbeur via une ligne contenant au moins une pompe de recirculation, ce premier adsorbeur servant d'unité de séparation (SMB-1)
- on connecte le dernier lit du deuxième adsorbeur au premier lit du deuxième adsorbeur via une ligne contenant au moins une pompe de recirculation, ce deuxième adsorbeur servant d'unité de séparation (SMB-2).

Dans une variante du procédé de production de paraxylène à haute pureté selon l'invention, la configuration des deux unités de séparation (SMB-1) et (SMB-2) est à nombre de lits fixes dans chacune des zones chromatographiques de chacune des deux unités de séparation (SMB-1) et (SMB-2).

Enfin, dans une autre variante du procédé de production de paraxylène à haute pureté selon l'invention, une fraction de l'isomérat (12) est envoyé à la colonne de distillation (S-1).

### DESCRIPTION DETAILLEE DE L'INVENTION

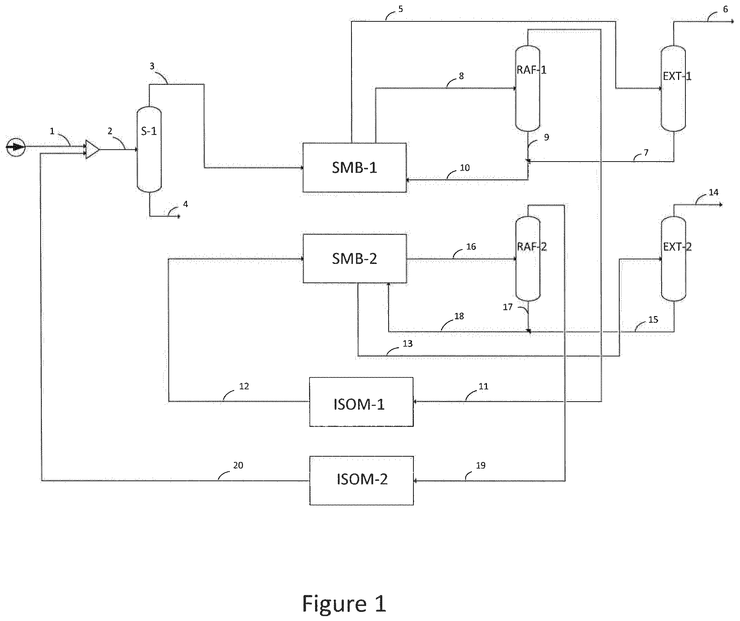

La présente description s'appuie sur la figure 1.

On envoie la charge (2) dans une colonne de distillation (S-1) d'où l'on soutire en tête un mélange (3) comprenant la majeure partie du métaxylène, du paraxylène, de l'éthylbenzène, et au moins une partie de l'orthoxylène, et d'où l'on soutire en fond un flux (4) d'hydrocarbures en C9-C10 et la partie restante d'orthoxylène.

On effectue une première séparation du mélange de tête (3) dans l'unité de séparation (SMB-1) dans au moins un adsorbeur contenant une pluralité de lits interconnectés et travaillant en boucle fermée, ledit adsorbeur comprenant au moins quatre zones délimitées par les injections du flux (3) constituant la première charge de la colonne et du désorbant (10), et les soutirages d'un extrait (5) contenant du paraxylène et d'un raffinat (8) contenant de l'orthoxylène et du métaxylène.

Les quatre zones correspondent aux définitions suivantes :
1) la zone 1 est comprise entre l'injection du désorbant (10) et le soutirage de l'extrait (5),
2) la zone 2 est comprise entre le soutirage de l'extrait (5) et l'injection de la première charge d'adsorption (3),
3) la zone 3 est comprise entre l'injection de la première charge (3) et le soutirage du raffinat (8) et,
4) la zone 4 est comprise entre le soutirage du raffinat (8) et l'injection du désorbant (10).
5)

On distille préférentiellement un premier extrait (5) dans une colonne à distiller (EXT-1), pour récupérer une première fraction (6) enrichie en paraxylène.

On distille préférentiellement un premier raffinat (8) dans une colonne à distiller (RAF-1) pour éliminer sensiblement tout le désorbant et pour soutirer une première fraction distillée (11). Cette première fraction distillée (11) alimente une première unité d'isomérisation (ISOM-1) pour obtenir un premier isomérat (12) alimentant préférentiellement l'unité de séparation des xylènes (SMB-2) mais pouvant être en partie recyclé en entrée de la colonne de distillation (S-1).

On effectue une deuxième séparation dans l'unité de séparation (SMB-2) de l'isomérat (12) issu de l'unité d'isomérisation (ISOM-1), dont on a optionnellement retiré tout ou partie des composés lourds en C9 et C10 par distillation (soit dans une colonne dédiée, soit dans la colonne S-1), l'unité de séparation (SMB-2) étant constituée d'au moins un adsorbeur contenant une pluralité de lits interconnectés, et travaillant préférentiellement en boucle fermée, ladite colonne comprenant au moins quatre zones délimitées par les injections du flux (12) et du désorbant (18), et les soutirages d'un deuxième extrait (13) contenant du paraxylène et d'un deuxième raffinat (16).

Les quatre zones se définissent de la façon suivante :
1) la zone 1 est comprise entre l'injection du désorbant (18) et le soutirage de l'extrait (13),
2) la zone 2 est comprise entre le soutirage de l'extrait (13) et l'injection de la deuxième charge d'adsorption (12),
3) la zone 3 est comprise entre l'injection de la charge (12) et le soutirage du raffinat (16), et
4) la zone 4 est comprise entre le soutirage du raffinat (16) et l'injection du désorbant (18).

On distille préférentiellement le deuxième extrait (13) dans une colonne de distillation (EXT-2), pour récupérer une deuxième fraction (14) enrichie en paraxylène. Les deux extraits (5) et (13) peuvent également être distillés dans une seule colonne d'extrait commune pour récupérer une seule fraction enrichie en paraxylène

On distille préférentiellement un deuxième raffinat (16) dans une colonne de distillation (RAF-2) pour éliminer sensiblement tout le désorbant (17) et pour soutirer une deuxième fraction distillée (19). Cette deuxième fraction distillée (19) alimente une deuxième unité d'isomérisation (ISOM-2) pour obtenir un deuxième isomérat (20) recyclé en entrée de la colonne de distillation (S-1).

La première unité d'isomérisation (ISOM-1) travaillant de préférence en phase liquide est généralement opérée dans les conditions suivantes :
- Température inférieure à 300°C, de préférence 200°C à 260°C
- Pression inférieure à 4 MPa, de préférence 2 à 3 MPa
- Vitesse spatiale horaire (VVH) inférieure à 10h⁻¹ (10 litres par litre et par heure), de préférence comprise entre 2 et 4 h⁻¹.
- Catalyseur comportant au moins une zéolithe présentant des canaux dont l'ouverture est définie par un anneau à 10 ou 12 atomes d'oxygène (10 MR ou 12 MR), préférentiellement un catalyseur comportant au moins une zéolithe présentant des canaux dont l'ouverture est définie par un anneau à 10 atomes d'oxygène (10 MR), et de manière encore plus préférée un catalyseur comportant une zéolithe de type ZSM-5.

La deuxième unité d'isomérisation (ISOM-2) est caractérisée en ce que le catalyseur comporte au moins une zéolithe présentant des canaux dont l'ouverture est définie par un anneau à 10 ou 12 atomes d'oxygène (10 MR ou 12 MR), préférentiellement un catalyseur comportant une zéolithe de type structural EUO ou MOR, et au moins un métal du groupe VIII de teneur comprise entre 0,1 et 0,3% pds bornes incluses, préférentiellement une zéolithe EU-1 et du platine. Cette zone d'isomérisation travaillant en phase gazeuse est généralement opérée dans les conditions suivantes :
- Température supérieure à 300°C, de préférence 350°C à 480°C
- Pression inférieure à 4 MPa, de préférence 0,5 à 2 MPa
- Vitesse spatiale horaire (VVH) inférieure à 10h⁻¹ (10 litres par litre et par heure), de préférence comprise entre 0,5 et 6 h⁻¹.
- Rapport molaire H₂/hydrocarbures inférieur à 10, de préférence compris entre 3 et 6.

Les désorbants utilisés dans les unités de séparation (SMB-1 et SMB-2) sont généralement choisis parmi le paradiéthylbenzène, le toluène, le paradifluorobenzène ou des diéthylbenzènes en mélange. Le rapport volumique du désorbant sur la charge dans les unités de séparation (SMB-1 et SMB-2) est compris entre 0,5 et 2,5, et de préférence compris entre 0,8 et 2.

Les unités de séparation (SMB-1 et SMB-2) sont opérées à une température comprise entre 20°C et 250°C, de préférence entre 90°C et 210 °C, et de manière encore préférée entre 140°C et 180°C, et sous une pression comprise entre la pression de bulle de xylènes à la température opératoire et 2 MPa.

Le fonctionnement du procédé selon l'invention, et en particulier la composition des différents flux, est précisé ci-après avec référence à la figure 1.

La charge fraîche est introduite par la ligne (1) dans une colonne à distiller (S-1).

Cette charge fraîche contient majoritairement des composés C8-aromatiques, xylènes et éthylbenzène, en proportion variable selon l'origine de la coupe. Elle peut contenir éventuellement des impuretés en quantité variable selon l'origine de la charge qui seront essentiellement des composés C9 et C10 aromatiques et des composés paraffiniques et naphténiques.

La teneur en impuretés naphténiques ou paraffiniques est avantageusement inférieure à 1 % poids. De manière préférée, cette teneur est inférieure à 0,3 % poids et de manière encore préférée cette teneur est inférieure à 0,1 % poids.

La charge peut être issue soit d'une unité de reformage, soit d'une unité de dismutation du toluène, soit d'une unité de transalkylation du toluène et des C9 aromatiques.

On ajoute à la charge fraîche un isomérat véhiculé par une ligne (20).

L'effluent de fond (4) de la colonne (S-1) est constitué essentiellement de composés en C9 et C10 aromatiques et éventuellement d'orthoxylène. Optionnellement, le mélange (4) d'orthoxylène et d'hydrocarbures aromatiques en C9-C10 soutiré en fond de la colonne de distillation (S1), peut être envoyé dans une autre colonne de distillation d'où l'on extrait en tête un flux d'orthoxylène de haute pureté (au moins 98,5 %), et en fond un flux contenant des hydrocarbures en C9-C10.

L'effluent de tête (3) de la colonne de distillation (S-1), constitue la charge de l'unité de séparation (SMB-1). L'unité de séparation (SMB-1) est alimentée d'une part par la charge véhiculée par la ligne (3), et d'autre part par du désorbant véhiculé par une ligne (10).

Les effluents de l'unité de séparation (SMB-1) sont un extrait (5) et un raffinat (8). Le nombre total de lits de l'unité de séparation (SMB-1) selon l'invention est de préférence compris entre 6 et 24 lits, et de manière encore plus préférée entre 8 et 15 lits répartis sur un ou plusieurs adsorbeurs.

Le nombre de lits sera ajusté de manière à ce que chaque lit ait de préférence une hauteur comprise entre 0,70 m et 1,40 m.

La répartition de la quantité de solide adsorbant dans chaque zone est la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%,

Selon une caractéristique préférée de l'invention, on peut injecter le désorbant et la charge dans l'unité de séparation dans un rapport volumétrique désorbant sur charge d'au plus 1,7/1, et de manière préférée compris entre 1,5/1 et 0,4/1, bornes incluses.

L'extrait (5) est constitué essentiellement de toluène, de paraxylène et de désorbant.

Le raffinat (8) est constitué essentiellement de toluène, de métaxylène, d'orthoxylène, d'éthylbenzène, de paraxylène pour la partie non récupéré dans l'extrait, et de désorbant. L'extrait (5) est envoyé dans une colonne à distiller (EXT-1).

On soutire de la colonne (EXT-1) le désorbant (7) qui est renvoyé dans l'unité de séparation (SMB-1) par la ligne (10), et un flux enrichie en paraxylène par la ligne (6).

Le raffinat (8) est envoyé dans une colonne à distiller (RAF-1). On soutire de la colonne à distiller (RAFF-1) du désorbant (9) qui est renvoyé dans l'unité de séparation (SMB-1) par la ligne (10), et un mélange de métaxylène, d'orthoxylène et d'éthylbenzène par une ligne (11), que l'on envoie vers l'unité d'isomérisation (ISOM-1).

L'unité d'isomérisation (ISOM-1), préférentiellement en phase liquide, peut travailler dans les conditions suivantes :
- température inférieure à 300°C, de préférence comprise entre 200 et 260°C,
- pression inférieure à 4 MPa, de préférence comprise entre 2 et 3 MPa,
- vitesse spatiale inférieure à 10 h⁻¹ de préférence comprise entre 2 h⁻¹ et 4 h⁻¹.

Tous les catalyseurs susceptibles d'isomériser les hydrocarbures à 8 atomes de carbone conviennent pour l'unité d'isomérisation (ISOM-1) de la présente invention. De préférence, on utilisera un catalyseur contenant une zéolithe de type ZSM-5.

L'effluent de l'unité d'isomérisation (ISOM-1) est renvoyé par la ligne (12), soit vers la colonne à distiller (S-1), soit directement à l'entrée de l'unité de séparation (SMB-2) dans le cas où la teneur en composés autres que les C8-aromatiques est très faible, typiquement de l'ordre de 1% poids. La teneur en C9 est typiquement inférieure à 1000 ppm pds.

Les effluents de l'unité de séparation (SMB-2) sont un extrait (13) et un raffinat (16).

Le nombre total de lits de l'unité de séparation (SMB-2) selon l'invention est de préférence compris entre 6 et 24 lits, et de manière encore plus préférée compris entre 8 et 15 lits répartis sur un ou plusieurs adsorbeurs.

Le nombre de lits sera ajusté de manière à ce que chaque lit ait de préférence une hauteur comprise entre 0,70 m et 1,40 m.

La répartition de la quantité de solide adsorbant dans chaque zone est préférentiellement la suivante :
- la quantité de solide adsorbant en zone 1 est de 17%±5%,
- la quantité de solide adsorbant en zone 2 est de 42%±5%,
- la quantité de solide adsorbant en zone 3 est de 25%±5%,
- la quantité de solide adsorbant en zone 4 est de 17%±5%,

Selon une caractéristique préférée de l'invention, on peut injecter le désorbant et la charge dans l'unité de séparation (SMB-2) dans un rapport volumétrique désorbant sur charge d'au plus 1,7/1, et de manière préférée compris entre 1,5/1 et 0,4/1, bornes incluses.

La configuration (nombre moyen de lits par zone) des deux unités de séparation (SMB-1 et SMB-2) peut être :
- à nombre de lits fixes dans chacune des zones chromatographiques (mode « lit mobile simulé » tel que défini dans le brevet FR2 976 501),
- à nombre de lits variable pour un adsorbeur (mode « VARICOL » tel que défini dans le brevet FR2 976 501), et fixe pour l'autre,
- à nombre de lits variable pour les deux adsorbeurs.

L'extrait (13) est constitué essentiellement de toluène, de paraxylène et de désorbant.

Le raffinat (16) est constitué essentiellement de toluène, de métaxylène et d'orthoxylène, d'éthylbenzène, de paraxylène pour la partie non récupéré dans l'extrait, et de désorbant. L'extrait (13) est envoyé dans une colonne à distiller (EXT-2).

On soutire de la colonne de distillation (EXT-2) le désorbant (15) qui est renvoyé dans l'unité de séparation (SMB-2) par la ligne (18), et un flux enrichie en paraxylène par la ligne (14).

Le raffinat est envoyé par une ligne (16) dans la colonne à distiller (RAF-2).

On soutire de de la colonne à distiller (RAF-2) le désorbant (17) qui est réintroduit par la ligne (18) dans l'unité de séparation (SMB-2), et un mélange de xylènes et d'éthylbenzène par une ligne (19).

Les effluents de la ligne (19) sont envoyés vers l'unité d'isomérisation (ISOM-2) fonctionnant à haute température en phase vapeur.

L'unité d'isomérisation (ISOM-2) est opérée de préférence dans les conditions suivantes :
- température supérieure à 300°C, de préférence de 360°C à 480°C,
- pression inférieure à 2,5 MPa et de préférence comprise entre 0,5 et 0,8 MPa,
- vitesse spatiale inférieure à 10 h⁻¹ de préférence comprise entre 0,5 h⁻¹ et 6 h⁻¹,
- rapport molaire hydrogène sur hydrocarbure inférieur à 10, et de préférence compris entre 3 et 6.

Tous les catalyseurs susceptibles d'isomériser les hydrocarbures à 8 atomes de carbone, zéolithiques ou non, conviennent pour l'unité d'isomérisation (ISOM-2) de la présente invention. De préférence, on utilise un catalyseur contenant une zéolithe acide, par exemple de type structural MFI, MOR, MAZ, FAU et/ou EUO. De manière encore plus préférée, on utilise un catalyseur contenant une zéolithe de type structural EUO et au moins un métal du groupe VIII de la classification périodique des éléments.

De manière préférée, le catalyseur de l'unité d'isomérisation (ISOM-2) renferme de 1 à 70% poids d'une zéolithe de type structural EUO (EU-1 par exemple) comprenant du silicium et au moins un élément T choisi de préférence parmi l'aluminium et le bore dont le rapport Si/T est compris entre 5 et 100. La dite zéolithe est sous forme hydrogène au moins en partie, et la teneur en sodium est telle que le ratio atomique Na/T est inférieur à 0,1. Eventuellement le catalyseur de l'unité d'isomérisation peut contenir entre 0,01 et 2% poids d'étain ou d'indium, et du soufre à raison de 0,5 à 2 atomes par atome de métal du groupe VIII.

L'effluent de l'unité d'isomérisation (ISOM-2) est envoyé dans un train de séparations qui permet de récupérer une partie de l'hydrogène que l'on recycle à l'unité d'isomérisation (ISOM-2). La partie d'hydrogène non recyclé est compensée par un appoint d'hydrogène frais. On récupère à l'issue du train de séparation un isomérat constitué des fractions les plus lourdes qui est renvoyé vers la colonne à distiller (S-1) par la ligne (20).

Le procédé selon l'invention est particulièrement bien adapté à une modification d'une unité existante en vue d'une augmentation de la quantité de paraxylène produite, opération appelée dégoulottage.

Dans le cas particulier d'un dégoulottage d'une boucle aromatique existante, l'invention consiste à augmenter de manière significative le débit de charge fraîche et le débit de paraxylène produit tout en continuant à utiliser les équipements principaux de la boucle soit :
1) la colonne de distillation des xylènes (S-1)
2) l'unité de séparation des xylènes en lit mobile simulé fonctionnant en 24 lits
3) l'unité d'isomérisation (ISOM-2) alimenté par le raffinat soutiré de la colonne de séparation convertissant l'éthylbenzène, ladite unité utilisant par exemple un catalyseur à base de zéolithe de type structural EUO comportant un réacteur, un compresseur de recyclage, une colonne de stabilisation, et une colonne permettant de récupérer les naphtènes en C8 et C9 de manière à les recycler à la charge,
4) la colonnes de raffinat (RAF-1)
5) la colonne d'extrait (EXT-1)

Pour réaliser ce dégoulottage selon l'invention, on transforme un procédé en lit mobile simulé à 24 lits contenant deux adsorbeurs de 12 lits en série, en un procédé en deux adsorbeurs de douze lits, chacun connecté en parallèle. Pour ce faire :
- on connecte le douzième lit du premier adsorbeur au premier lit dudit premier adsorbeur via une ligne contenant au moins une pompe de recirculation,
- on connecte le douzième lit du deuxième adsorbeur au premier lit dudit deuxième adsorbeur via une ligne contenant au moins une pompe de recirculation.

Le système de contrôle et de régulation des débits d'injection de charge et de désorbant et des débits de soutirage de l'extrait et du raffinat de l'étape d'adsorption en 24 lits est adapté de manière à pouvoir gérer indépendamment les débits d'injection et de soutirage dans chacun des deux adsorbeurs du procédé remodelé selon l'invention.

Pour les dispositifs d'injection, cette opération pourra être réalisée soit en doublant le système pompe + organe de mesure pour réguler le débit injecté dans chacun des adsorbeurs, soit, dans le but de minimiser les coûts, en utilisant la pompe et l'organe de mesure préexistants qui gèreront l'ensemble des deux flux à injecter, et en ajoutant un système de mesure et de régulation du débit alimentant l'un des deux adsorbeurs.

Lorsque l'alimentation ou le soutirage des fluides sur l'ensemble des plateaux du procédé existant en 24 lits est assuré par une pluralité de vannes commandées tout ou rien, il n'y a pas de modifications supplémentaires à apporter aux réseaux d'alimentation et de soutirage.

Lorsque l'alimentation ou le soutirage des fluides sur l'ensemble des plateaux du procédé existant en 24 lits est assurée par l'emploi d'une vanne rotative multi-voies, ces fonctions seront de manière préférée assurées par l'utilisation de deux vannes rotatives multi-voies 15 (en recyclant éventuellement la vanne préexistante sur l'une des deux voies après adaptation).

Dans le cas d'une unité existante en 24 lits constituée de deux fois douze lits en série, le flux principal circule du fond du premier adsorbeur vers la tête du second adsorbeur, et du fond du second adsorbeur vers la tête du premier adsorbeur.

Les flux issus des fonds des deux adsorbeurs sont alors réorientés pour circuler vers la tête de l'adsorbeur dont ils sont issus en réalisant les modifications de vannes et de conduites. Le flux de fond du premier adsorbeur est recyclé vers la tête dudit adsorbeur et le flux de fond du deuxième adsorbeur est recyclé vers la tête dudit deuxième adsorbeur.

La configuration (nombre moyen de lits par zone) des deux adsorbeurs peut être réalisée selon l'une des 3 variantes exposées plus haut c'est-à-dire :
- à nombre de lits fixes dans chacune des zones chromatographiques pour les deux adsorbeurs,
- à nombre de lits variable pour un adsorbeur et fixe pour l'autre,
- à nombre de lits variable pour les deux adsorbeurs.

De manière à séparer le deuxième raffinat du désorbant, une nouvelle colonne à distiller (RAF-2) devra également être mise en place. Le flux de xylènes et d'éthylbenzène soutiré de la colonne à distiller (RAF-2) sera isomérisée dans l'unité d'isomérisation en phase vapeur (ISOM-1) telle que décrite plus haut.

On ajoute une deuxième unité d'isomérisation en phase liquide (ISOM-2) dont l'effluent alimentera l'unité de séparation (SMB-2) préférentiellement sans passer par la colonne de distillation (S-1) pour éviter l'ajout d'une deuxième colonne de séparation des xylènes.

D'autre part, il faut rajouter une colonne de distillation d'extrait (EXT-2) alimentée par l'extrait (13) de l'unité de séparation (SMB-2).

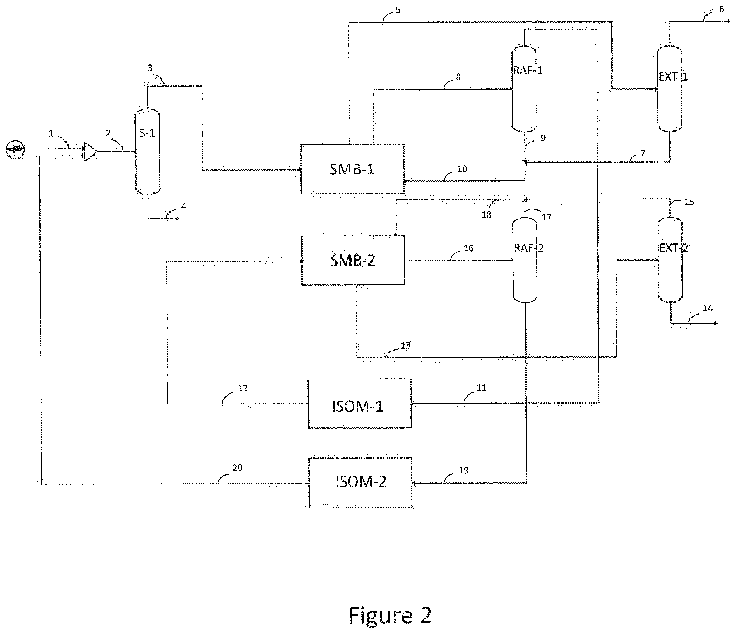

La figure 2 représente une variante du schéma de procédé selon l'invention qui diffère de celui de la figure 1 par le fait que l'unité de séparation (SMB-1) utilise le PDEB comme désorbant et le toluène comme désorbant de l'unité de séparation (SMB-2).

Contrairement à l'unité de séparation (SMB-1) au PDEB, l'unité de séparation (SMB-2) récupère le désorbant (toluène) en tête de colonne EXT-2 et RAF-2, car le toluène est plus léger que les C8-aromatiques.

Le fait d'utiliser deux désorbants différents dans les deux unités de séparation (SMB-1 et SMB-2) présente l'avantage d'éviter l'accumulation des impuretés aromatiques telles que le benzène et les composés aromatiques lourds en C9 et C10.

Utiliser deux désorbants différents dans les deux unités de séparation (SMB-1 et SMB-2) permet également de réaliser une intégration thermique entre les colonnes (EXT-1) et (EXT-2) et (RAF-1) et (RAF-2). En effet la tête de colonne (EXT-1) peut potentiellement rebouillir tout ou partie de la colonne (EXT-2) et la tête de la colonne (RAF-1) peut potentiellement rebouillir tout ou partie de la colonne (RAF-2).

### EXEMPLES SELON L'INVENTION

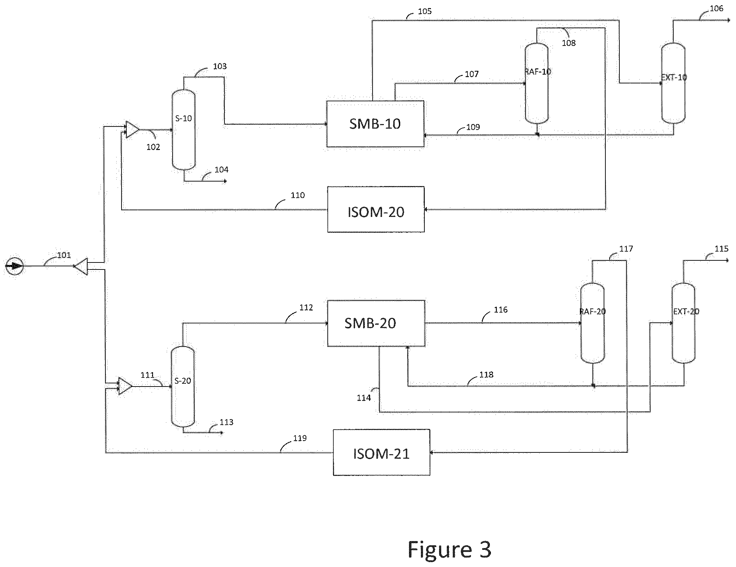

### Exemple 1 selon l'art antérieur (figure 3) :

Cet exemple illustre l'art antérieur et décrit un complexe aromatique constitué de deux boucles C8-aromatique en parallèle, typiques des complexes industriels dans lesquels la quantité de paraxylène produite est supérieure à la capacité acceptable par une boucle C8-aromatique unique, tel que schématisé sur la figure 3 et comportant :
- deux colonne des xylènes (S-10 et S-20) permettant d'extraire les aromatiques en C9 et C10 (flux 104 et 113) et d'envoyer aux unités de séparation (SMB-10 et SMB-20) un flux (103) et un flux (112) constitué essentiellement d'aromatiques en C8,
- une première unité de séparation en lit mobile simulé (SMB-10) à 4 zones de laquelle on soutire un extrait (105) et un unique raffinat (107),
- une première unité d'isomérisation (ISOM-10) alimentée par une partie (108) du raffinat (107) après élimination du désorbant (109) au moyen de la colonne à distiller (RAF-10),
- une première colonne d'extrait du paraxylène (EXT-10) dont on soutire en fond le désorbant qu'on recycle à l'unité de séparation (SMB-1) via le flux (109) et en tête une coupe riche en paraxylène (106),
- une deuxième unité de séparation en lit mobile simulé (SMB-20) à 4 zones de laquelle on soutire un extrait (114) et un unique raffinat (116),
- une deuxième unité d'isomérisation (ISOM-20) alimentée par une partie (117) du raffinat (116) après élimination du désorbant (118) au moyen de la colonne à distiller (RAF-20),
- une deuxième colonne d'extrait du paraxylène (EXT-20) dont on soutire en fond le désorbant qu'on recycle à l'adsorption (SMB-2) via le flux (118) et en tête une coupe riche en paraxylène (115).

Le bilan matière du procédé est décrit dans le tableau ci-dessous. Seuls les composés C8-aromatiques et C9+ sont décrits. On néglige les autres composés et la formation de C9+ dans les unités d'isomérisation. On utilise comme unité de débit le millier de tonnes par an (kt/an).

**TABLEAU 1**

| | | PX | EB | MOX | C9+ | Total |
|---|---|---|---|---|---|---|
| Charge fraîche | 101 | 23,6 | 15,6 | 67,7 | 13,8 | 120,6 |
| Charge S-10 | 102 | 50,0 | 22,9 | 148,5 | 6,9 | 228,3 |
| Charge SMB-10 | 103 | 50,0 | 22,9 | 148,5 | 0 | 221,4 |
| Fond S-10 | 104 | 0 | 0 | 0 | 6,9 | 6,9 |
| Tête EXT-10 | 106 | 50,0 | 0 | 0 | 0 | 50,0 |
| Entré ISOM-10 | 108 | 0 | 22,9 | 148,5 | 0 | 171,4 |
| Sortie ISOM-10 | 110 | 38,2 | 15,1 | 114,7 | 0 | 168,0 |
| Charge S-20 | 111 | 50,0 | 22,9 | 148,5 | 6,9 | 228,3 |
| Charge SMB-20 | 112 | 50,0 | 22,9 | 148,5 | 0 | 221,4 |
| Fond S-20 | 113 | 0 | 0 | 0 | 6,9 | 6,9 |
| Tête EXT-20 | 115 | 50,0 | 0 | 0 | 0 | 50,0 |
| Entré ISOM-20 | 117 | 0 | 22,9 | 148,5 | 0 | 171,4 |
| Sortie ISOM-20 | 119 | 38,2 | 15,1 | 114,7 | 0 | 168,0 |

La charge (101) qui alimente la boucle aromatique (mélange du réformat lourd et du fond de la colonne de toluène) présente un débit de 120,6 kt/an. Cette charge est divisée en deux flux égaux de 60,3 kt/an.

On adjoint à une première partie de la charge (101) 168 kt/an d'isomérat (110) recyclés de l'unité d'isomérisation (ISOM-10) de manière à isomériser l'éthylbenzène.

Le flux résultant (102) est distillé dans la colonne de xylènes (S-10).

On soutire en fond de la colonne (S-10) 6,9 kt/an d'un mélange de C9 et C10 aromatiques (104) et en tête 221,4 kt/an d'une coupe C8 aromatiques (103) dont la teneur en paraxylène est de 22,6%, la teneur en éthylbenzène est de 10,3%, la teneur en orthoxylène et métaxylène est de 67,1%.

Cette coupe est envoyée dans une unité de séparation en lit mobile simulé à quatre zones (SMB-10) et quatre flux principaux : la charge (103), le désorbant (109), l'extrait (105) et le raffinat (107). Cette unité est composée de 12 lits contenant une zéolithe X échangée au baryum. La température est de 175°C. La configuration est : 2 lits en zone 1, 5 lits en zone 2, 3 lits en zone 3 et 2 lits en zone 4. Le solvant utilisé est le paradiéthylbenzène.

L'extrait (105) en sortie de l'unité de séparation (SMB-10) est envoyé dans une colonne à distiller (EXT-10) de laquelle on tire en fond le désorbant recyclé vers l'unité de séparation (SMB-10), et en tête 50 kt/an d'un mélange (106) essentiellement constitué de toluène et de paraxylène.

Le raffinat est envoyé dans une colonne à distiller (RAF-10) de laquelle on tire en fond le désorbant recyclé vers l'unité de séparation (SMB-10), et en tête 171,4 kt/an d'un mélange (108).

Ce flux est envoyé dans une unité d'isomérisation (ISOM-10).

L'unité d'isomérisation (ISOM-10) travaille en phase gazeuse aux conditions suivantes :
Température : 385°C
Catalyseur : contient 0,2%pds de platine et de la zéolithe EU-1
Vitesse spatiale : 3,5 h⁻¹
Rapport H₂/hydrocarbures : 4,4 :1
Pression : 0,9 MPa

La teneur en éthylbenzène du mélange introduit dans l'unité d'isomérisation (ISOM-10) est de 13,4%.

On observe 2% de perte par craquage dans cette isomérisation soit un débit de 3,4 kt/an. L'éthylbenzène est isomérisé en partie, il en reste 9% dans le flux de sortie (110).

Cet isomérat (110) présente un débit de 168 kt/an. Il est recyclé en entrée de la colonne S-10 où il est mélangé avec une partie de la charge fraîche (101) qui présente un débit de 60,3 kt/an.

On adjoint à une première partie de la charge (101) 168 kt/an d'isomérat (119) recyclés de l'unité d'isomérisation (ISOM-20) de manière à isomériser l'éthylbenzène. Le flux résultant (111) est distillé dans la colonne de xylènes (S-20).

On soutire en fond de la colonne à distiller (S-20) 6,9 kt/an d'un mélange de C9 et C10 aromatiques (flux 113) et en tête 221,4 kt/an de coupe C8 aromatiques (flux 112) dont la teneur en paraxylène est de 22,6%, et la teneur en éthylbenzène de 10,4%.

Cette coupe est envoyée dans une unité d'adsorption en lit mobile simulé à quatre zones (SMB-20) et quatre flux principaux : la charge (flux 112), le désorbant (flux 118), l'extrait (flux 114) et le raffinat (flux 116).

Cette unité est composée de 12 lits contenant une zéolithe X échangée au baryum.

La température est de 175°C. La configuration est : 2 lits en zone 1, 5 lits en zone 2, 3 lits en zone 3 et 2 lits en zone 4. Le solvant utilisé est le paradiéthylbenzène.

L'extrait (114) en sortie de l'unité de séparation (SMB-20) est envoyé dans une colonne à distiller (EXT-20) de laquelle on tire en fond le désorbant recyclé vers l'unité d'adsorption (SMB-20), et en tête 50 kt/an d'un mélange (115) essentiellement constitué de toluène et de paraxylène.

Le raffinat est envoyé dans une colonne à distiller (RAF-20) de laquelle on tire en fond le désorbant recyclé vers l'unité de séparation (SMB-20), et en tête 171,4 kt/an d'un mélange (117).

Ce flux est envoyé dans une unité d'isomérisation (ISOM-20). L'isomérat obtenu (119) est recyclé en entrée de la colonne de distillation (S-20) où il est mélangé avec une partie de la charge fraîche (101).

L'unité d'isomérisation (ISOM-20) travaille en phase gazeuse aux conditions suivantes :
Température : 385°C
Catalyseur : contient 0,2%pds de platine et de la zéolithe EU-1
Vitesse spatiale : 3,5 h⁻¹
Rapport H₂/hydrocarbures : 4,4/1
Pression : 0,9 MPa

La teneur en éthylbenzène du mélange introduit dans l'unité d'isomérisation (ISOM-20) est de 13,4%.

On observe 2% de perte par craquage dans cette isomérisation soit un débit de 3,4 kt/an. L'éthylbenzène est isomérisé en partie, il en reste 9% dans le flux de sortie (119).

Cet isomérat (119) présente un débit de 168 kt/an, il est recyclé en entrée de la colonne S-20 où il est mélangé avec une partie de la charge fraîche (101) qui présente un débit de 60,3 kt/an.

### Exemple 2 selon l'invention :

Cet exemple illustre l'invention et décrit une boucle aromatique schématisée sur la figure 1 et comportant :
- une colonne des xylènes (S-1) permettant d'extraire les aromatiques en C9 et C10 (4) et d'envoyer à l'unité d'adsorption (SMB-1) un flux (3) constitué essentiellement d'aromatiques en C8.
- une première unité de séparation en lit mobile simulé (SMB-1) à 4 zones de laquelle on soutire un extrait (5) et un raffinat (8).
- une première colonne d'extrait du paraxylène (EXT-1) dont on soutire en fond le désorbant (7) qu'on recycle à l'unité de séparation (SMB-1) via le flux (10) et en tête une coupe riche en paraxylène (6).
- une deuxième unité de séparation en lit mobile simulé (SMB-2) à 4 zones de laquelle on soutire un extrait (13) et un raffinat (16).
- une deuxième colonne d'extrait du paraxylène (EXT-2) dont on soutire en fond le désorbant (15) qu'on recycle à l'unité de séparation (SMB-2) via le flux (18) et en tête une coupe riche en paraxylène (14).
- une première unité d'isomérisation (ISOM-1) alimentée par le premier raffinat (11) après élimination du désorbant (9) au moyen de la colonne à distiller (RAF-1).
- une deuxième unité d'isomérisation (ISOM-2) alimentée par le deuxième raffinat (19) après élimination du désorbant (17) au moyen de la colonne à distiller (RAF-2).

Le bilan matière du procédé est décrit dans le tableau 2 ci-dessous. Seuls les composés C8- et C9+ sont décrits, on néglige les autres composés et la formation de C9+ dans les unités d'isomérisation. On utilise comme unité de débit le millier de tonnes par an (kt/an).

**TABLEAU 2**

| | | PX | EB | MOX | C9 | Total |
|---|---|---|---|---|---|---|
| Charge fraîche | 1 | 22,7 | 15,3 | 65,1 | 13,3 | 116,4 |
| Charge S-1 | 2 | 57,6 | 29,1 | 169,7 | 13,3 | 269,7 |
| Charge SMB-1 | 3 | 57,6 | 29,1 | 169,7 | 0 | 256,4 |
| Fond S-1 | 4 | 0 | 0 | 0 | 13,3 | 13,3 |
| Tête EXT-1 | 6 | 57,6 | 0 | 0 | 0 | 57,6 |
| Charge ISOM-1 | 11 | 0 | 29,1 | 169,7 | 0 | 198,8 |
| Sortie ISOM-1 | 12 | 42,4 | 29,1 | 127,3 | 0 | 198,8 |
| Tête EXT-2 | 14 | 42,4 | 0 | 0 | 0 | 42,4 |
| Entrée ISOM-2 | 19 | 0 | 29,1 | 127,3 | 0 | 156,4 |
| Sortie ISOM-2 | 20 | 34,9 | 13,8 | 104,6 | 0 | 153,3 |

La charge fraîche (1) qui alimente la boucle aromatique présente un débit de 116,4 kt/an. On adjoint à cette charge 153,3 kt/an d'isomérat (20) recyclés de l'unité d'isomérisation (ISOM-2) isomérisant l'éthylbenzène. Le flux résultant (2) est distillé dans la colonne de xylènes (S-1).

On soutire en fond de la colonne à distiller (S-1) 13,3 kt/an d'un mélange de C9 et C10 aromatiques (4), et en tête 256,4 kt/an de coupe C8 aromatiques (3) dont la teneur en paraxylène est de 22,5%, la teneur en éthylbenzène de 11,3%, la teneur en orthoxylène et en métaxylène de 66,2%.

Cette coupe en C8 aromatiques est envoyée dans une unité de séparation en lit mobile simulé à quatre zones (SMB-1) et quatre flux principaux : la charge (3), le désorbant (10), l'extrait (5) et le raffinat (8). Cette unité est composée de 12 lits contenant une zéolithe X échangée au baryum. La température est de 175°C. La configuration est : 2 lits en zone 1, 5 lits en zone 2, 3 lits en zone 3 et 2 lits en zone 4.

Le solvant utilisé est le paradiéthylbenzène.

L'extrait (5) en sortie de l'unité de séparation (SMB-1) est envoyé dans une colonne à distiller (EXT-1) de laquelle on tire en fond le désorbant (7) recyclé vers l'unité de séparation (SMB-1), et en tête 57,6 kt/an d'un mélange (6) essentiellement constitué de toluène et de paraxylène.

Le raffinat (8) est envoyé dans une colonne à distiller (RAF-1) de laquelle on tire en fond le désorbant (9) recyclé vers l'unité d'adsorption (SMB-1), et en tête 198,8 kt/an d'un mélange (11).

Ce flux (11) est envoyé dans une unité d'isomérisation (ISOM-1).

L'unité d'isomérisation (ISOM-1) travaille en phase liquide aux conditions suivantes :
Température : 240°C
Catalyseur : contient de la zéolithe ZSM-5
Vitesse spatiale : 3 h⁻¹
Pression : 1,9 MPa

La teneur en éthylbenzène du mélange introduit dans l'unité d'isomérisation (ISOM-1) est environ 14,6%.

L'éthylbenzène n'étant pas converti, sa quantité est donc la même dans le flux de sortie (12).

L'isomérat (12) présente un débit de 198,8 kt/an. Il est recyclé en entrée de l'unité d'adsorption (SMB-2) sans passer par la colonne (S-1).

L'isomérat (12) issu de l'unité d'isomérisation (ISOM-1) alimente une deuxième unité de séparation en lit mobile simulé à quatre zones (SMB-2) et quatre flux principaux : la charge (12), le désorbant (18), l'extrait (13) et le raffinat (16).

Cette unité est composée de 12 lits contenant une zéolithe X échangée au baryum.

La température est de 175°C. La configuration est : 2 lits en zone 1, 5 lits en zone 2, 3 lits en zone 3 et 2 lit en zone 4. Le solvant utilisé est le paradiéthylbenzène.

L'extrait (13) en sortie de l'unité de séparation (SMB-2) est envoyé dans une colonne à distiller (EXT-2) de laquelle on tire en fond le désorbant (15) recyclé vers l'unité de séparation (SMB-2), et en tête 42,4 kt/an d'un mélange (14) essentiellement constitué de toluène et de paraxylène.

Le raffinat (16) est envoyé dans une colonne à distiller (RAF-2) de laquelle on tire en fond le désorbant (17) recyclé vers l'unité de séparation (SMB-2), et en tête 156,4 kt/an d'un mélange (19).

Ce flux (19) est envoyé dans une unité d'isomérisation (ISOM-2).

L'unité d'isomérisation (ISOM-2) travaille en phase gaz aux conditions suivantes :
Température : 385°C
Catalyseur : contient 0,2%pds de platine et de la zéolithe EU-1
Vitesse spatiale : 3,5 h⁻¹
Pression : 0,9 MPa

La teneur en éthylbenzène du mélange introduit dans l'unité d'isomérisation (ISOM-2) est environ 18,6%. On observe 2% de perte par craquage dans cette unité d' isomérisation, soit un débit de 3,1 kt/an.

L'éthylbenzène est isomérisé en partie, il en reste 9% dans le flux de sortie (20).

L'isomérat (20) présente un débit de 153,3 kt/an, il est recyclé en entrée de la colonne S-1 où il est mélangé avec la charge fraîche (1) qui présente un débit de 116,4 kt/an.

L'invention présente plusieurs avantages par rapport à l'art antérieur.

Tout d'abord l'unité d'isomérisation en phase liquide est moins énergivore que l'isomérisation en phase gaz.

En effet, elle travaille à température plus faible. Elle travaille également sans recycle d'hydrogène donc sans compresseur de recycle.

Enfin, l'unité d'isomérisation en phase liquide produit beaucoup moins de sous-produits, notamment des aromatiques en C9, ce qui permet de by passer la colonne d'élimination des aromatiques en C9 (S-1), induisant une très forte baisse de l'énergie nécessaire à cette séparation. Le fait de coupler une isomérisation en phase liquide à une isomérisation en phase gaz isomérisant l'éthylbenzène, permet de diminuer les pertes par craquage.

En effet, pour sortir 100 kt/an de paraxylène, il faut introduire 103,1 kt/an de composés aromatiques en C8 (calculé comme la différence 116,4 - 13,3 kt/an) dans la charge fraîche dans le procédé selon l'invention, contre 106,8 kt/an de composés aromatiques en C8 (calculé comme la différence 120,6 -13,8 kt/an) pour le procédé selon l'art antérieur.

## Revendications

1. Procédé de production de paraxylène à haute pureté, à partir d'une coupe xylènes contenant de l'éthylbenzène et des composés en C9+, procédé utilisant deux unités de séparation en lit mobile simulé (SMB-1 et SMB-2), et deux unités d'isomérisation (ISOM-1 et ISOM-2) consistant en la suite d'étapes suivantes :
- on envoie la charge (2) dans une colonne de distillation (S-1), de laquelle on soutire en tête un mélange (3) comprenant la majeure partie du métaxylène, du paraxylène, de l'éthylbenzène, et au moins une partie de l'orthoxylène, et en fond un flux (4) d'hydrocarbures en C9-C10, et la partie restante de l'orthoxylène,
- on effectue une première séparation du mélange de tête (3) dans l'unité de séparation (SMB-1) comportant au moins un adsorbeur contenant une pluralité de lits interconnectés et travaillant en boucle fermée, ladite unité comprenant au moins quatre zones délimitées par les injections du flux (3) et du désorbant (10) résultant du mélange des flux (9) et (7), et les soutirages d'un premier extrait (5) enrichi en paraxylène, et d'un premier raffinat (8) appauvri en paraxylène,
- on effectue une deuxième séparation dans l'unité de séparation (SMB-2) de l'isomérat (12) issu de l'unité d'isomérisation (ISOM-1), ladite unité de séparation (SMB-2) étant constituée d'au moins un adsorbeur contenant une pluralité de lits interconnectés et travaillant préférentiellement en boucle fermée, et ladite unité comprenant au moins quatre zones délimitées par les injections de la charge (12) et du désorbant (18) résultant des flux (17) et (15), et les soutirages d'un deuxième extrait (13) enrichi en paraxylène, et d'un second raffinat (16) appauvri en paraxylène,
- on distille le premier extrait (5) issu de l'unité de séparation (SMB-1) dans une colonne à distiller (EXT-1), pour récupérer un flux (6) enrichi en paraxylène, et un flux (7) qui est utilisé comme désorbant de l'unité de séparation (SMB-1),
- on distille le second extrait (13) issu de l'unité de séparation (SMB-2) dans une colonne à distiller (EXT-2), pour récupérer un flux (14) enrichi en paraxylène, et un flux (15) qui est utilisé comme désorbant de l'unité de séparation (SMB-2),
- on distille le raffinat (16) issu de l'unité de séparation (SMB-2) dans une colonne de distillation (RAF-2), de manière à produire un flux (19) qui alimente l'unité d'isomérisation (ISOM-2), et un flux (17) qui est utilisé comme désorbant de l'unité de séparation (SMB-2),
- on distille le raffinat (8) issu de l'unité de séparation (SMB-1) dans une colonne à distiller (RAF-1) qui produit le flux (11) qui alimente l'unité d'isomérisation (ISOM-1), et un flux (9) qui est utilisé comme désorbant de l'unité de séparation (SMB-1),
- on alimente par le flux (11) l'unité d'isomérisation (ISOM-1) pour obtenir le premier isomérat (12),
- on alimente par le flux (19) la deuxième unité d'isomérisation (ISOM-2) pour obtenir un deuxième isomérat (20) qui est recyclé en entrée de la colonne à distiller (S-1), ladite unité d'isomérisation (ISOM-2) fonctionnant en phase gaz et aux conditions suivantes :
- température supérieure à 300°C, de préférence de 350°C à 480°C,
- pression inférieure à 4,0 MPa, et de préférence de 0,5 à 2,0 MPa,
- vitesse spatiale inférieure à 10 h⁻¹, de préférence comprise entre 0,5 h⁻¹ et 6 h⁻¹,
- rapport molaire hydrogène sur hydrocarbure inférieur à 10, et de préférence compris entre 3 et 6,
et le catalyseur utilisé dans ladite unité d'isomérisation (ISOM-2) comportant au moins une zéolithe présentant des canaux dont l'ouverture est définie par un anneau à 10 ou 12 atomes d'oxygène (noté 10 MR ou 12 MR), et au moins un métal du groupe VIII de teneur comprise entre 0,1 et 0,3% poids, bornes incluses.

2. Procédé de production de paraxylène à haute pureté à partir d'une coupe xylènes contenant de l'éthylbenzène et des composés en C9+ selon la revendication 1, dans lequel l'unité d'isomérisation (ISOM1) fonctionne en phase liquide aux conditions suivantes :
- Température inférieure à 300°C, de préférence comprise entre 200 et 260°C,
- Pression inférieure à 4 MPa, de préférence comprise entre 2 et 3 MPa,
- Vitesse spatiale inférieure à 10 h⁻¹, de préférence comprise entre 2 h⁻¹ et 4 h⁻¹,
- Catalyseur comportant au moins une zéolithe présentant des canaux dont l'ouverture est définie par un anneau à 10 ou 12 atomes d'oxygène (noté 10 MR ou 12 MR), préférentiellement une zéolithe présentant des canaux dont l'ouverture est définie par un anneau à 10 atomes d'oxygène (noté 10 MR), et de manière encore plus préférée une zéolithe de type ZSM-5.

3. Procédé de production de paraxylène à haute pureté à partir d'une coupe xylènes contenant de l'éthylbenzène et des composés en C9+ selon la revendication 1, dans lequel le catalyseur utilisé pour l'unité d'isomérisation en phase gaz (ISOM-2) contient une zéolithe de type structural EUO ou MOR, et de manière préférée, une zéolithe EU-1 et du platine.

4. Procédé de production de paraxylène à haute pureté à partir d'une coupe xylènes contenant de l'éthylbenzène et des composés en C9+ selon la revendication 1, dans lequel l'unité de séparation (SMB-1) utilise comme désorbant le PDEB.

5. Procédé de production de paraxylène à haute pureté à partir d'une coupe xylènes contenant de l'éthylbenzène et des composés en C9+ selon la revendication 1, dans lequel l'unité de séparation (SMB-2) utilise comme désorbant le toluène.

6. Procédé de production de paraxylène à haute pureté à partir d'une coupe xylènes contenant de l'éthylbenzène et des composés en C9+ selon la revendication 1, dans lequel les unités de séparation (SMB-1) et (SMB-2) contiennent chacune de 6 à 24 lits, et de manière préférée de 8 à 15 lits répartis sur un ou plusieurs adsorbeurs, le nombre de lits étant ajusté de manière à ce que chaque lit ait de préférence une hauteur comprise entre 0,70 m et 1,40 m.

7. Procédé de production de paraxylène à haute pureté à partir d'une coupe xylènes contenant de l'éthylbenzène et des composés en C9+ selon la revendication 1, dans lequel la répartition de la quantité de solide adsorbant dans les unités de séparation (SMB-1) et (SMB-2) est la suivante :
• la quantité de solide adsorbant en zone 1 est de 17%±5%,
• la quantité de solide adsorbant en zone 2 est de 42%±5%,
• la quantité de solide adsorbant en zone 3 est de 25%±5%,
• la quantité de solide adsorbant en zone 4 est de 17%±5%,
les zones étant définies de la façon suivante :
- la zone 1 étant comprise entre l'injection du désorbant et le soutirage de l'extrait,
- la zone 2 étant comprise entre le soutirage de l'extrait et l'injection de la charge,
- la zone 3 étant comprise entre l'injection de la charge et le soutirage du raffinat,
- la zone 4 étant comprise entre le soutirage du raffinat et l'injection du désorbant.

8. Procédé de production de paraxylène à haute pureté à partir d'une coupe xylènes contenant de l'éthylbenzène et des composés en C9+ selon la revendication 1, dans lequel pour l'unité de séparation (SMB-1), le rapport volumétrique désorbant sur charge est d'au plus 1,7/1, et de manière préférée compris entre 1,5/1 et 0,4/1, bornes comprises.

9. Procédé de production de paraxylène à haute pureté à partir d'une coupe xylènes contenant de l'éthylbenzène et des composés en C9+ selon la revendication 1, dans lequel pour l'unité de séparation (SMB-2), le rapport volumétrique désorbant sur charge est d'au plus 1,7/1, et de manière préférée compris entre 1,5/1 et 0,4/1 bornes comprises.

10. Procédé de production de paraxylène à haute pureté à partir d'une coupe xylènes contenant de l'éthylbenzène et des composés en C9+ selon la revendication 1, procédé obtenu à partir du dégoulottage d'une unité existante, constituée de deux adsorbeurs utilisés en série, de la manière suivante :
- on connecte le dernier lit du premier adsorbeur au premier lit du premier adsorbeur via une ligne contenant au moins une pompe de recirculation, ce premier adsorbeur servant d'unité de séparation (SMB-1)
- on connecte le dernier lit du deuxième adsorbeur au premier lit du deuxième adsorbeur via une ligne contenant au moins une pompe de recirculation, ce deuxième adsorbeur servant d'unité de séparation (SMB-2).

11. Procédé de production de paraxylène à haute pureté à partir d'une coupe xylènes contenant de l'éthylbenzène et des composés en C9+ selon la revendication 1, dans lequel la configuration des deux unités de séparation (SMB-1) et (SMB-2) est à nombre de lits fixes dans chacune des zones chromatographiques de chacune des deux unités de séparation (SMB-1) et (SMB-2).

12. Procédé de production de paraxylène à haute pureté à partir d'une coupe xylènes contenant de l'éthylbenzène et des composés en C9+ selon la revendication 1, dans lequel une fraction de l'isomérat (12) est envoyé à la colonne de distillation (S-1).

## Patentansprüche

1. Verfahren zur Herstellung von hochreinem Paraxylol aus einem Xylolschnitt, der Ethylbenzol und [C9+]-Verbindungen enthält, wobei das Verfahren zwei simulierte Wanderbetttrennungseinheiten (SMB-1 und SMB-2) und zwei Isomerisierungseinheiten (ISOM-1 und ISOM-2) verwendet, bestehend aus der folgenden Reihe von Schritten:
- Einleiten des Einsatzmaterials (2) in eine Destillationssäule (S-1), aus der am Kopf eine Mischung (3), die den Großteil des Metaxylols, Paraxylol, Ethylbenzol und mindestens einen Teil des Orthoxylols aufweist, und am Boden ein Strom (4) von C9-C10-Kohlenwasserstoffen und der verbleibende Teil des Orthoxylols entnommen wird,
- Durchführen einer ersten Trennung der Kopfmischung (3) in der Trennungseinheit (SMB-1), die mindestens einen Adsorber aufweist, der mehrere miteinander verbundene Betten enthält und in einem geschlossenen Kreislauf arbeitet, wobei die Einheit mindestens vier Zonen aufweist, die durch die Einspritzungen des Stroms (3) und des Desorbens (10), die aus der Mischung der Ströme (9) und (7) resultieren, und den Entnahmen eines ersten Extrakts (5), der mit Paraxylol angereichert ist, und eines ersten Raffinats (8), das an Paraxylol abgereichert ist, begrenzt sind,
- Durchführen einer zweiten Trennung in der Trennungseinheit (SMB-2) des Isomerats (12), das aus der Isomerisierungseinheit (ISOM-1) stammt, wobei die Trennungseinheit (SMB-2) aus mindestens einem Adsorber besteht, der mehrere miteinander verbundene Betten enthält und vorzugsweise in einem geschlossenen Kreislauf arbeitet, und wobei die Einheit mindestens vier Zonen aufweist, die durch die Einspritzungen des Einsatzmaterials (12) und des Desorbens (18), die aus den Strömen (17) und (15) resultieren, und den Entnahmen eines zweiten Extrakts (13), der mit Paraxylol angereichert ist, und eines zweiten Raffinats (16), das an Paraxylol abgereichert ist, begrenzt sind,
- Destillieren des ersten Extrakts (5), der aus der Trennungseinheit (SMB-1) stammt, in einer Destillationskolonne (EXT-1), um einen Strom (6), der mit Paraxylol angereichert ist, und einen Strom (7), der als Desorbens der Trennungseinheit (SMB-1) verwendet wird, zu gewinnen,
- Destillieren des zweiten Extrakts (13), der aus der Trennungseinheit (SMB-2) stammt, in einer Destillationskolonne (EXT-2), um einen Strom (14), der mit Paraxylol angereichert ist, und einen Strom (15), der als Desorbens der Trennungseinheit (SMB-2) verwendet wird, zu gewinnen,
- Destillieren des Raffinats (16), das aus der Trennungseinheit (SMB-2) stammt, in einer Destillationskolonne (RAF-2), um einen Strom (19), mit dem die Isomerisierungseinheit (ISOM-2) gespeist wird, und einen Strom (17), der als Desorbens der Trennungseinheit (SMB-2) verwendet wird, herzustellen,
- Destillieren des Raffinats (8), das aus der Trennungseinheit (SMB-1) stammt, in einer Destillationskolonne (RAF-1), die einen Strom (11), mit dem die Isomerisierungseinheit (ISOM-1) gespeist wird, und einen Strom (9), der als Desorbens der Trennungseinheit (SMB-1) verwendet wird, herstellt,
- Einspeisen des Stroms (11) in die Isomerisierungseinheit (ISOM-1), um das erste Isomerat (12) zu erhalten,
- Einspeisen des Stroms (19) in die zweite Isomerisierungseinheit (ISOM-2), um ein zweites Isomerat (20) zu erhalten, das an den Einlass der Destillationskolonne (S-1) zurückgeführt wird, wobei die Isomerisierungseinheit (ISOM-2) in der Gasphase und unter den folgenden Bedingungen arbeitet:
- Temperatur oberhalb von 300 °C, vorzugsweise von 350 °C bis 480 °C,
- Druck unterhalb von 4,0 Mpa und vorzugsweise von 0,5 bis 2,0 MPa,
- Raumgeschwindigkeit von unter 10 h⁻¹, vorzugsweise im Bereich zwischen 0,5 h⁻¹ und 6 h⁻¹,
- Molverhältnis von Wasserstoff zu Kohlenwasserstoff kleiner 10 und bevorzugt im Bereich zwischen 3 und 6, und wobei der in der Isomerisierungseinheit (ISOM-2) verwendete Katalysator mindestens einen Zeolithen mit Kanälen, deren Öffnung durch einen Ring mit 10 oder 12 Sauerstoffatomen definiert ist (als 10 MR oder 12 MR bezeichnet), und mindestens ein Metall der Gruppe VIII in einem Anteil im Bereich zwischen 0,1 und 0,3 Gew.-%, einschließlich der Grenzen, aufweist.

2. Verfahren zur Herstellung von hochreinem Paraxylol aus einem Xylolschnitt, der Ethylbenzol und [C9+]-Verbindungen enthält, nach Anspruch 1, wobei die Isomerisierungseinheit (ISOM1) in der flüssigen Phase unter den folgenden Bedingungen arbeitet:
- Temperatur unterhalb von 300 °C, vorzugsweise im Bereich zwischen 200 und 260 °C,
- Druck unterhalb von 4 MPa, vorzugsweise im Bereich zwischen 2 und 3 MPa,
- Raumgeschwindigkeit von unter 10 h⁻¹, vorzugsweise im Bereich zwischen 2 h⁻¹ und 4 h⁻¹,
- Katalysator, der mindestens einen Zeolithen mit Kanälen, deren Öffnung durch einen Ring mit 10 oder 12 Sauerstoffatomen definiert ist (als 10 MR oder 12 MR bezeichnet), vorzugsweise einen Zeolithen mit Kanälen, deren Öffnung durch einen Ring mit 10 Sauerstoffatomen definiert ist (als 10 MR bezeichnet), und noch stärker bevorzugt einen Zeolithen vom ZSM-5-Typ aufweist.

3. Verfahren zur Herstellung von hochreinem Paraxylol aus einem Xylolschnitt, der Ethylbenzol und [C9+]-Verbindungen enthält, nach Anspruch 1, wobei der für die Isomerisierungseinheit in der Gasphase (ISOM-2) verwendete Katalysator einen Zeolithen vom Strukturtyp EUO oder MOR und vorzugsweise einen EU-1-Zeolithen und Platin, enthält.

4. Verfahren zur Herstellung von hochreinem Paraxylol aus einem Xylolschnitt, der Ethylbenzol und [C9+]-Verbindungen enthält, nach Anspruch 1, wobei die Trennungseinheit (SMB-1) PDEB als Desorbens verwendet.

5. Verfahren zur Herstellung von hochreinem Paraxylol aus einem Xylolschnitt, der Ethylbenzol und [C9+]-Verbindungen enthält, nach Anspruch 1, wobei die Trennungseinheit (SMB-2) Toluol als Desorbens verwendet.

6. Verfahren zur Herstellung von hochreinem Paraxylol aus einem Xylolschnitt, der Ethylbenzol und [C9+]-Verbindungen enthält, nach Anspruch 1, wobei die Trennungseinheiten (SMB-1) und (SMB-2) jeweils 6 bis 24 Betten und vorzugsweise 8 bis 15 Betten enthalten, die auf einem oder mehreren Adsorbern verteilt sind, wobei die Anzahl der Betten so angepasst ist, dass jedes Bett vorzugsweise eine Höhe im Bereich zwischen 0,70 m und 1,40 m hat.

7. Verfahren zur Herstellung von hochreinem Paraxylol aus einem Xylolschnitt, der Ethylbenzol und [C9+]-Verbindungen enthält, nach Anspruch 1, wobei die Verteilung der Menge an Feststoffadsorbens in den Trennungseinheiten (SMB-1) und (SMB-2) wie folgt ist:
• die Menge an Feststoffadsorbens in der Zone 1 beträgt 17 %±5 %,
• die Menge an Feststoffadsorbens in der Zone 2 beträgt 42 %±5 %,
• die Menge an Feststoffadsorbens in der Zone 3 beträgt 25 %±5 %,
• die Menge an Feststoffadsorbens in der Zone 4 beträgt 17 %±5 %,
wobei die Zonen wie folgt definiert sind:
- die Zone 1 liegt im Bereich zwischen der Desorbenseinspritzung und der Extraktentnahme,
- die Zone 2 liegt im Bereich zwischen der Extraktentnahme und der Einsatzmaterialeinspritzung,
- die Zone 3 liegt im Bereich zwischen der Einsatzmaterialeinspritzung und der Raffinatentnahme,
- die Zone 4 liegt im Bereich zwischen der Raffinatentnahme und der Desorbenseinspritzung.

8. Verfahren zur Herstellung von hochreinem Paraxylol aus einem Xylolschnitt, der Ethylbenzol und [C9+]-Verbindungen enthält, nach Anspruch 1, wobei für die Trennungseinheit (SMB-1) das volumetrische Verhältnis von Desorbens zum Einsatzmaterial bei höchsten 1,7/1, und bevorzugt im Bereich zwischen 1,5/l und 0,4/l, einschließlich der Grenzen, liegt.

9. Verfahren zur Herstellung von hochreinem Paraxylol aus einem Xylolschnitt, der Ethylbenzol und [C9+]-Verbindungen enthält, nach Anspruch 1, wobei für die Trennungseinheit (SMB-2) das volumetrische Verhältnis von Desorbens zum Einsatzmaterial bei höchsten 1,7/1, und bevorzugt im Bereich zwischen 1,5/l und 0,4/l, einschließlich der Grenzen, liegt.

10. Verfahren zur Herstellung von hochreinem Paraxylol aus einem Xylolschnitt, der Ethylbenzol und [C9+]-Verbindungen enthält, nach Anspruch 1, wobei das Verfahren aus dem Debottlenecking einer bestehenden Einheit, die aus zwei in Reihe geschalteten Adsorbern besteht, wie folgt erhalten wird:
- Verbinden des letzten Betts des ersten Adsorbers mit dem ersten Bett des ersten Absorbers über eine Leitung, die mindestens eine Umlaufpumpe enthält, wobei dieser erste Adsorber als Trennungseinheit (SMB-1) dient
- Verbinden des letzten Betts des zweiten Adsorbers mit dem ersten Bett des zweiten Absorbers über eine Leitung, die mindestens eine Umlaufpumpe enthält, wobei dieser zweite Adsorber als Trennungseinheit (SMB-2) dient.

11. Verfahren zur Herstellung von hochreinem Paraxylol aus einem Xylolschnitt, der Ethylbenzol und [C9+]-Verbindungen enthält, nach Anspruch 1, wobei die Konfiguration der beiden Trennungseinheiten (SMB-1) und (SMB-2) eine feste Anzahl an Betten in jeder der Chromatographiezonen jeder der beiden Trennungseinheiten (SMB-1) und (SMB-2) aufweist.

12. Verfahren zur Herstellung von hochreinem Paraxylol aus einem Xylolschnitt, der Ethylbenzol und [C9+]-Verbindungen enthält, nach Anspruch 1, wobei eine Fraktion des Isomerats (12) in die Destillationskolonne (S-1) geleitet wird.

## Claims

1. Process for the production of high-purity paraxylene based on a xylenes cut containing ethylbenzene and C9+ compounds, a process using two simulated moving bed separation units (SMB-1 et SMB-2), and two isomerization units (ISOM-1 et ISOM-2) consisting of the following series of stages:
- the feedstock (2) is sent into a distillation column (S-1) from which a mixture (3) is drawn off at the top comprising the major part of the metaxylene, paraxylene, ethylbenzene, and at least a part of the orthoxylene, and a flow (4) of C9-C10 hydrocarbons and the remaining part of the orthoxylene is drawn off at the bottom.
- a first separation of the mixture from the top (3) is carried out in the separation unit (SMB-1) comprising at least one adsorber containing a plurality of interconnected beds and operating in a closed loop, said unit comprising at least four zones delimited by the injections of the flow (3) and of the desorbent (10), resulting from the mixture of the flows (9) and (7), and the draw-offs of a first extract (5) enriched with paraxylene, and of a first raffinate (8) depleted of paraxylene,
- a second separation of the isomerate (12) originating from the isomerization unit (ISOM-1) is carried out in the separation unit (SMB-2), said separation unit (SMB-2) being constituted by at least one adsorber containing a plurality of interconnected beds and operating preferentially in a closed loop, and said unit comprising at least four zones delimited by the injections of the feedstock (12) and of the desorbent (18) resulting from the flows (17) and (15), and the draw-offs of a second extract (13) enriched with paraxylene, and of a second raffinate (16) depleted of paraxylene,
- the first extract (5) originating from the separation unit (SMB-1) is distilled in a distillation column (EXT-1), in order to recover a flow (6) enriched with paraxylene, and a flow (7) which is used as desorbent of the separation unit (SMB-1),
- the second extract (13) originating from the separation unit (SMB-2) is distilled in a distillation column (EXT-2), in order to recover a flow (14) enriched with paraxylene, and a flow (15) which is used as desorbent of the separation unit (SMB-2),
- the raffinate (16) originating from the separation unit (SMB-2) is distilled in a distillation column (RAF-2), so as to produce a flow (19) which supplies the isomerization unit (ISOM-2), and a flow (17) which is used as desorbent of the separation unit (SMB-2),
- the raffinate (8) originating from the separation unit (SMB-1) is distilled in a distillation column (RAF-1) which produces the flow (11) which supplies the isomerization unit (ISOM-1), and a flow (9) which is used as desorbent of the separation unit (SMB-1),
- the isomerization unit (ISOM-1) is supplied with the flow (11), in order to obtain the first isomerate (12),
- the second isomerization unit (ISOM-2) is supplied with the flow (19), in order to obtain a second isomerate (20), which is recycled to the inlet of the distillation column (S-1), said isomerization unit (ISOM-2) operating in gas phase and under the following conditions.
- temperature greater than 300°C, preferably from 350°C to 480°C,
- pressure less than 4.0 MPa and preferably from 0.5 to 2.0 MPa,
- hourly space velocity less than 10 h⁻¹, preferably comprised between 0.5 h⁻¹ and 6 h⁻¹,
- hydrogen to hydrocarbon molar ratio less than 10, and preferably comprised between 3 and 6, and the catalyst used in said isomerization unit (ISOM-2) comprising at least one zeolite having channels the opening of which is defined by a ring with 10 to 12 oxygen atoms (denoted 10 MR or 12 MR), and at least one group VIII metal at a content comprised between 0.1 and 0.3% by weight, inclusive.

2. Process for the production of high-purity paraxylene based on a xylenes cut containing ethylbenzene and C9+ compounds according to claim 1, in which the isomerization unit (ISOM-1) operates in liquid phase under the following conditions:
- Temperature less than 300°C, preferably comprised between 200 and 260°C,
- Pressure less than 4MPa, preferably comprised between 2 and 3 MPa,
- Hourly space velocity less than 10 h⁻¹, preferably comprised between 2 h⁻¹ and 4 h⁻¹,
- Catalyst comprising at least one zeolite having channels the opening of which is defined by a ring with 10 or 12 oxygen atoms (denoted 10 MR or 12 MR), preferentially a catalyst comprising at least one zeolite having channels the opening of which is defined by a ring with 10 oxygen atoms (denoted 10 MR), and even more preferably, a catalyst including a zeolite of the ZSM-5 type.

3. Process for the production of high-purity paraxylene based on a xylenes cut containing ethylbenzene and C9+ compounds according to claim 1, in which the catalyst used for the gas-phase isomerization unit (ISOM-2) contains a zeolite of the EUO or MOR structure type and, preferably, an EU-1 zeolite and platinum.

4. Process for the production of high-purity paraxylene based on a xylenes cut containing ethylbenzene and C9+ compounds according to claim 1, in which the separation unit (SMB-1) uses PDEB as desorbent.

5. Process for the production of high-purity paraxylene based on a xylenes cut containing ethylbenzene and C9+ compounds according to claim 1, in which the separation unit (SMB-2) uses toluene as desorbent.

6. Process for the production of high-purity paraxylene based on a xylenes cut containing ethylbenzene and C9+ compounds according to claim 1, in which the separation units (SMB-1) and (SMB-2) each contain from 6 to 24 beds, and preferably from 8 to 15 beds, distributed over one or more adsorbers, the number of beds being adjusted so that each bed preferably has a height comprised between 0.70 m and 1.40 m.

7. Process for the production of high-purity paraxylene based on a xylenes cut containing ethylbenzene and C9+ compounds according to claim 1, in which the distribution of the quantity of solid adsorbent in the separation units (SMB-1) and (SMB-2) is as follows:
• the quantity of solid adsorbent in zone 1 is 17%±5%,
• the quantity of solid adsorbent in zone 2 is 42%±5%,
• the quantity of solid adsorbent in zone 3 is 25%±5%,
• the quantity of solid adsorbent in zone 4 is 17%±5%,
the zones being defined as follows:
- zone 1 being comprised between the injection of the desorbent and the draw-off of the extract,
- zone 2 being comprised between the draw-off of the extract and the injection of the feedstock,
- zone 3 being comprised between the injection of the feedstock and the draw-off of the raffinate,
- zone 4 being comprised between the draw-off of the raffinate and the injection of the desorbent.

8. Process for the production of high-purity paraxylene based on a xylenes cut containing ethylbenzene and C9+ compounds according to claim 1, in which, for the separation unit (SMB-1), the ratio by volume of desorbent to feedstock is at least 1.7/1 and preferably comprised between 1.5/1 and 0.4/1, inclusive.

9. Process for the production of high-purity paraxylene based on a xylenes cut containing ethylbenzene and C9+ compounds according to claim 1, in which, for the separation unit (SMB-2), the ratio by volume of desorbent to feedstock is at least 1.7/1 and preferably comprised between 1.5/1 and 0.4/1, inclusive.

10. Process for the production of high-purity paraxylene from a xylenes cut containing ethylbenzene and C9+ compounds according to claim 1, a process obtained based on the debottlenecking of an existing separation unit, constituted by two adsorbers used in series, as follows:
- the last bed of the first adsorber is connected to the first bed of the first adsorber via a line containing at least one recirculation pump, this first adsorber acting as a separation unit (SMB-1)
- the last bed of the second adsorber is connected to the first bed of the second adsorber via a line containing at least one recirculation pump, this second adsorber acting as a separation unit (SMB-2).

11. Process for the production of high-purity paraxylene based on a xylenes cut containing ethylbenzene and C9+ compounds according to claim 1, in which the configuration of the two separation units (SMB-1) and (SMB-2) has a fixed number of beds in each of the chromatographic zones of each of the two separation units (SMB-1 and SMB-2).

12. Process for the production of high-purity paraxylene based on a xylenes cut containing ethylbenzene and C9+ compounds according to claim 1, in which a fraction of the isomerate (12) is sent to the distillation column (S-1)
